# EUROPEAN PATENT APPLICATION

(11) **EP 4 691 395 A1**
(43) Date of publication of application: **11.02.2026**
(21) Application number: 24723608.6
(22) Date of filing: 01.04.2024
(51) Int. Cl.: A61B 17/80

(54) **DEVICE FOR CORRECTING ROTATIONAL MALALIGNMENT SYNDROME**

(30) Priority: 30.03.2023 US 202363455667 P
(71) Applicant: Russi, Martin, Montevideo (UY); Olmedo, Santiago, Lot 24 20001 La Barra, Department of Maldonado (UY)
(72) Inventor: RUSSI, Martin, 2974 Montevideo (UY)
(74) Representative: Pons IP
(86) International application number: PCT/IB2024/053152
(87) International publication number: WO 2024/201429

(57) **Abstract**

The invention relates to a device for correction in the rotational plane of the axis of long bones during the growth push thereof, wherein the device comprises a fixing plate and a guide-stop to generate the relative rotation of bone zones where the plate and the guide-stop are respectively fixed, the invention also refers to a minimally invasive technique and positioning guide for the placement of said device.

## Description

### STATE OF THE ART OF THE INVENTION

### Field of invention

The present invention relates to the field of medical devices and, in particular, refers to a fixing plate and guide-stop device to generate the relative rotation of bone areas where the plate and the guide-stop are respectively fixed, preferably for correction in the rotational plane of the axis of long bones during the growth push thereof, the invention also refers to a minimally invasive placement technique of said device.

### Description of previous art

In the field of orthopedics, particularly in infant orthopedics, there exists what is known as rotational malalignment syndrome of the lower limbs that presents itself as a marked abnormality, with excessive medial torsion at femur level and excessive lateral rotation of the tibia. The affected individuals, when standing, place their knees in a deviated inward position and their feet in maximum external rotation.

Alterations in alignment can be constitutional, also known as physiological, or pathological. In general, the constitutional alterations occur in normal individuals, i.e., with normal development, without dysplastic signs, normal height, etc., and the deformity tends to be moderate, in many cases bilateral and symmetrical. In imaging tests such as simple x-ray (XR) or computerized axial tomography (CAT), no changes other than the deformity itself are detected. They have no known cause. They do not usually cause functional problems, but when they are severe, they produce aesthetic problems, gait alterations and mechanical imbalances that favor premature joint degeneration. An example of these would be the femoral anteversion or the genu valgum of development. Secondly, there are other alterations in alignment caused by pathological causes, whether congenital or acquired. Any pathological osteoarticular process of traumatic, neoplastic, inflammatory, infectious or degenerative origin will be susceptible to cause a deformity of the lower limbs. The treatment of these deformities will be dual: treating the specific cause that provokes it and the deformity itself.

Most children and adults walk with their feet in an average 10° external rotation. An internal rotation gait greater than 10° or external rotation gait greater than 30° is considered pathological. These deformities are usually originated at the level of femur or tibia, and are diagnosed by a physician, thanks to their knowledge on the development of the extremities of children, along with a good clinical evaluation, thus differentiating what is common, from what is pathological.

Rotational malalignment is a common problem in children. At an early age it often appears physiologically and it is often corrected with the growth of the child. Oftentimes, when the deformity is very marked or when it is not corrected with age, the only clear solution at the moment is osteotomy of the involved bone and derotation with internal fixation.

Derotation osteotomy surgery is an invasive surgery associated with several risks, such as bleeding, non-consolidation, displacement, incomplete correction or overcorrection, and risks associated with the implant being used, such as implant breakage or failure, associated infection. It is also a surgery that involves hospitalization, delayed weight load and costs for the health system.

For deformities in other planes, the coronal one above all, but also the sagittal one, the use of guided growth devices has long been proven and well accepted, which use the remaining growth of the child and modulate it in order to correct the alterations that are present.

Guided growth proposed by Professor Dr. Peter Stevens (plate in 8), for the correction of angular defects in the frontal and sagittal planes has demonstrated a minimally invasive solution without the need for bone cutting, a faster rehabilitation and the possibility of determining a transient procedure and can reverse physeal arrest, that is to say, of the physis, when the desired correction is achieved.

The so-called long bones exhibit at their end an area called epiphysis, which continues toward the center in another area called metaphysis after which, and continuing toward the center of the length of the bone, the body or diaphysis occurs. Between the epiphysis E and metaphysis M is located the physis fi which is the growth cartilage. The growth cartilage is important for bone development.

Recent studies have established the possibility of using this concept for the correction of rotational deformities, i.e., of the axial plane. If achieved, a less invasive technique, which avoid osteotomies and their associated complications, could be achieved.

In the last 10 years the idea of using guided growth in the axial plane has emerged for rotational correction of the immature skeleton.

The first author to begin investigating the possible use of a rotational guided growth system was Arami, who, in 2013, made a hypothesis that the placement of plates in an oblique arrangement, with both epiphyseal and metaphyseal tethering, would cause a subsequent rotation. This author produced a mathematical model and then made testing on rabbits, with a rigid plate, achieving the desired angular correction and theorizing that this concept could be used in the future. Remarkably, associated with rotational correction there occurs a shortening which could be considered a complication.

After this publication two more can be found which study different aspects of this rotational guided growth generated with oblique rigid plates in rabbits. Cobanoglu in 2016 studied 45 rabbits, evaluating whether this method managed to generate a rotation associated with the growth of these animals. Lazarus et al in 2017 delves a little further into the study of this construct and evaluates the angle of the placement of the plate and its relationship with the generated rotation.

Seeking to advance the generation of knowledge in this field, Martel scales in 2018 to studies in large animals, in particular calves, and proposes a different method to generate rotation: a construct with two cannulated screws, one metaphyseal and the other epiphyseal, and a circular wire that passes through both and is arranged with opposite obliquity in the lateral and medial sectors of the distal femur. He shows results that evidence the clear generation of a rotation associated with the growth of the animals with subsequent alignment of the placed screws.

More recently, and based on the previous studies mentioned, two studies have been carried out in humans.

In 2019, Metaizeau published a series of cases using a construct similar to those of Martel in the distal femur of 20 knees, showing a very good correction and establishing it as a possible valid alternative to derotation osteotomies. In the most recent study, from 2023, Paley published a series of cases using an implant with a principle similar to oblique plates but with less rigidity due to having an intermediate sector with very high strength tapes that employ a long chain polyethylene structure, known under the brand name Fibertape; surgeries were performed to correct both intra and extrarotation, and both in tibia and femur, also publishing hopeful results.

The system proposed by Paley in this analyzed study uses the two male hemiplates of the hinge plate system developed by the Pegamedical firm fixed with screw one to the metaphyseal sector and another to the epiphyseal sector, attached with Fibertape suture from Artrhex at a 45° angle in the internal sector and another in the external sector of the bone. See EFORT Open Reviews (2024) 9 119-128 https://doi.org/10.1530/EOR-23-0149, GENERAL ORTHOPAEDICS, "Correction of rotational deformities in long bones using guided growth: a scoping review" Ahmed Halloum 1, Søren Kold1, Jan Duedal Rölfing 2, Ahmed A Abood1,2 and Ole Rahbek1.

All the systems analyzed so far to generate a rotational correction during bone growth, that is to say, the rotational guided growth, propose a construct with an epiphyseal and metaphyseal tethering, so that after achieving the desired correction it must be removed, so as not to generate physeal arrest.

More precisely, in order to provoke the corrective rotation, i.e., the rotation between the bone and the epiphyseal plate, plates, bars and wires are fixed, with one end fixed to the plate and the opposite end fixed to the metaphysis, extending diagonally, so that, when the bone grows, the diagonal plate or wire causes the relative rotation between the bone and the epiphysis. However, after obtaining the rotational correction, the tethering, be it a plate, wire, etc., must be removed to prevent the tethering from slowing down the axial growth of the bone, that is to say, prevent the growth of the metaphysis.

The surgical technique used in these works is not analyzed in detail or they present difficult steps for the correct positioning of the proposed constructs, these placement techniques being fundamental for these systems to work correctly.

Even though these new techniques are promising, it is still necessary to improve their functioning avoiding having to return to surgery to untether and remove the element that causes the rotation, at best, just before starting to function as an arrest of growth.

### SUMMARY OF THE INVENTION

It is therefore an object of the present invention to provide a new tethering plate system for rotational correction between bone and epiphysis that works freely without risk of causing arrest of growth once the syndrome being treated is corrected or partially corrected.

It is another object of the present invention to provide a rotational corrective plate system that does not generate physeal arrest during guided growth and does not necessarily require a second surgery to release the system, or if it is necessary to continue the correction in the rotary plane, it can continue working with small surgical gestures.

It is another object of the present invention to provide a plate with epiphyseal tethering and metaphyseal extension that presents at least one hole at the epiphyseal level to generate a bone tethering which can comprise a cannulated blocked screw or not.

It is another object of the present invention to provide a plate with epiphyseal tethering and metaphyseal extension that presents at least one hole at the epiphyseal level to generate a bone tethering which can comprise a cannulated unblocked screw or not.

It is another object of the present invention to provide a plate with epiphyseal tethering and metaphyseal extension that presents at least one hole at the epiphyseal level to generate a bone tethering which can comprise a double-block head for Kirschner that allows attachment with the hole of contralateral symmetric plate.

**It** is another object of the present invention to provide a plate with epiphyseal tethering and metaphyseal extension, wherein the metaphyseal extension generates a fixed angle with the physis, which can be variable at the time of its manufacture, being preferably 60°.

**It** is another object of the present invention to provide a plate with epiphyseal tethering and metaphyseal extension, wherein the metaphyseal extension generates a fixed angle with the physis, which can be variable at the time of its manufacture and allows it to slide with respect to a metaphyseal stop that is placed.

**It** is another object of the present invention to provide a plate with epiphyseal tethering and metaphyseal extension, wherein the metaphyseal extension generates a fixed angle with the physis, which can be variable at the time of its manufacture, wherein said metaphyseal extension may be solid or it may present a complete or incomplete central recess to accommodate a metaphyseal stop that is placed allowing its sliding.

**It** is another object of the present invention to provide a plate with epiphyseal tethering and metaphyseal extension, wherein the metaphyseal extension generates a fixed angle with the physis, which can be variable at the time of its manufacture, said plate can be made of titanium, surgical steel or any other biocompatible material which can also be resorbable.

**It** is another object of the present invention to provide a bicortical metaphyseal element, which can be a screw or a bar that allows to generate a stop in the plate or plates that are placed, for which it must protrude from one or two cortical elements respectively.

**It** is another object of the present invention to provide a bicortical metaphyseal element, which can be a screw or a bar that allows to generate a stop in the plate or plates that are placed, which can be made of titanium, surgical steel, or any other biocompatible material, even in bioabsorbable material.

**It** is another object of the present invention to provide a bicortical metaphyseal element, which can be a cannulated screw or not, completely or partially threaded, with a self-tapping, self-piercing or blunt tip, which can generate a stop in the plate or plates that are placed, for which it must protrude from one or two cortical elements respectively, this element you can have an associated threaded washer or not on each side of the screw to create a lateral stop and prevent the system from being released, as well as cover the tip of the screw to avoid injury of the soft parts or discomfort to the patient.

**It** is another object of the present invention to provide a bicortical metaphyseal element, a cannulated bar or not, partially, or completely threaded, with blunt tips and the tips can be shaped as an Allen key and can be placed on or removed from either side, which can generate a stop on the plates to be placed, for which it must protrude from one or two cortical elements respectively.

**It** is another object of the present invention to provide a mono or bicortical metaphyseal element, which can be a screw or similar which can generate a stop in the ipsilateral plate, for which it must protrude from said cortical element and necessarily two of these components must be placed if a double plate is used.

**It** is another object of the present invention to provide a mono or bicortical metaphyseal element, which can be a screw or similar which can generate a stop in the ipsilateral plate, for which it must protrude from said cortical element and that necessarily two of these components must be placed if a double plate is used, made of titanium, surgical steel, or any biocompatible element, which can be resorbable or not.

**It** is another object of the present invention to provide a metaphyseal anchoring element, which can generate a stop in the metaphyseal extension of the ipsilateral plate, for which it must protrude from said cortical element and necessarily 2 of these components must be placed if a double plate is used. They can be tubular systems of different morphologies that allow the metaphyseal extension of the plates and are fixed to the bone in different ways.

**It** is another object of the present invention to provide a metaphyseal anchoring element, which can generate a stop in the metaphyseal extension of the ipsilateral plate, for which it must protrude from said cortical element and necessarily two of these components must be placed if a double plate is used. They can be staples of different morphologies that allow the metaphyseal extension of the plates and are fixed to the bone in different ways.

**It** is another object of the present invention to provide a metaphyseal anchoring element, which can generate a stop in the metaphyseal extension of the ipsilateral plate, for which it must protrude from said cortical element and necessarily two of these components must be placed if a double plate is used. They can be tubular systems of different morphologies that allow the metaphyseal extension of the plates and are fixed to the bone in different ways or they are staple-like systems, which can be made of titanium, surgical steel, or any biocompatible material, which can be resorbable or not.

**It** is another object of the present invention to provide a metaphyseal anchoring element, which can generate a stop in the metaphyseal extension of the plates, comprised of a cannulated screw that protrudes from both cortical layers, which is then threaded by a cerclage wire or cable that surrounds the metaphyseal extension of the plate that is in contact with the tip of the screw and this cerclage is fixed to the head of the screw.

**It** is another object of the present invention to provide a metaphyseal anchoring element, which can generate a stop in the metaphyseal extension of the plates, comprised of a cannulated screw that protrudes from both cortical parts, which is then threaded by a cerclage wire or cable that surrounds the metaphyseal extensions of the plates and this cerclage is fixed to the head of the screw.

**It** is an object of the present invention to provide a device for the correction of rotational malalignment syndrome in an individual suffering the syndrome, wherein the device comprises at least one fixing plate having an first end portion of the tethering having means of fixation for fixing it to a first zone of a bone, and a second free end portion designed to extend over a second area of the bone without being fixed to it, at least a guide-stop designed to be fixed to that second area of the bone, and at least one guiding edge in that second free end portion for guided support against said guide-stop.

**It** is yet another object of the present invention to provide a positioning guide for the installation of the device of the invention, comprising an internal part and an external part, movable grom one another to get closer or apart, wherein each of the internal and external parts present a sector designed to accommodate the shape of the fixing plate of the invention, said sector comprising two cylinders for the threading of installation wires, these sectors being fixed to respective arms that in turn join the telescopic coupling arms together.

**It** is another object of the present invention to provide a method of placement of the above positioning guide, comprising the steps of:
Step 1: Threading, through one of the cylinders of the internal and external parts of the guide respectively, a metaphyseal Kirschner wire previously positioned in the bone;
Step 2: assembling the arms that are attached telescopically;
Step 3: positioning of the placement guide in the profile, using the image intensifier to place said one of the cylinders, which is a radiopaque epiphyseal hollow cylinder, in the center of the epiphysis, which allows to place the epiphyseal Kirschner wire in its correct position, and then removing the guide.

**It** is another object of the present invention to provide a method of installation of the device of the invention, comprising the following steps:
Step 1: placing a metaphyseal Kirschner wire parallel to the physis E on the front of the bone, for example, 1 cm at metaphyseal level and in the center of the bone in the profile, seeking correct positioning with the use of the image intensifier;
Step 2: assembling the placement guide on the Kirschner wire, through the cylinder, which allows to locate the correct positioning of the epiphyseal Kirschner wire;
Step 3: placing an epiphyseal Kirschner wire through the guide and then removing the guide;
Step 4: milling with a cannulated bit through the metaphyseal wire and placing a bicortical cannulated screw, from the side in front of the plate, so that at the time of the rotation it is positioned upstream and facilitates its removal, positioning the bicortical screw so that it protrudes half a centimeter on each side of the bone, to generate the metaphyseal guide-stop;
Step 5: performing an epiphyseal approach of 3 cm, internal and external, centered on the Kirschner wire for the correct sliding of the epiphyseal plate, sliding the plate into contact with the second end portion or metaphyseal extension, with the guide-stop already installed, the sliding of the plate can be facilitated by placing a threaded guide sleeve in the central locking hole and thus using it as a handle;
Step 6: removing the epiphyseal Kirschner wire, positioning the plate with the centralizer hole where the Kirschner wire was located and milling through the locking sleeve, placing the locked centralizer screw through the plate, then placing the second locked screw on each side, closing it by planes performing a correct washing and hemostasis and controlling with an image intensifier the final positioning of the device.

### BRIEF DESCRIPTION OF THE DRAWINGS

For greater clarity and understanding of the object of the present invention, it has been illustrated in several figures, in which the invention has been represented in one of the preferred embodiments, all by way of example, wherein:
Figure 1 is a sagittal view of a left distal femur in which a pate has been implanted according to an embodiment of the present invention;
Figure 2 is a sagittal view of a left distal femur in which two plates, an internal one and an external one, have been implanted, according to another embodiment of the present invention;
Figure 3 is an axial view of a left distal femur in which two plates, an internal one and an external one, have been implanted, according to the invention;
Figure 4 is a sagittal view of a distal left femur, with internal and external mirroring plates to generate an external rotation of the distal femur;
Figure 5 shows a sagittal view of a left proximal tibia with a plate according to another embodiment of the invention;
Figure 6 shows a sagittal view of a left proximal tibia with a double plate according to another embodiment of the invention;
Figure 7 is a sagittal view of a distal left femur, with a plate according to another embodiment of the invention;
Figure 8 is a sagittal view of a distal left femur, with a plate according to another embodiment of the invention;
Figure 9 shows a view of two plates in combination with a cannulated screw, according to another embodiment of the invention;
Figure 10 shows a view of two plates in combination with a bicortical bar, according to another embodiment of the invention;
Figure 11 shows a view of two plates in combination with two screws as a metaphyseal stop, according to another embodiment of the invention;
Figure 12 shows a view of two plates in combination with two staples as a metaphyseal stop, according to another embodiment of the invention;
Figure 13 shows a view of two plates in combination with two sleeves as a metaphyseal stop, according to another embodiment of the invention;
Figure 14 shows a perspective view of a positioning guide used in the implant technique according to another embodiment of the invention, and
Figure 15 shows a sagittal view of a variant of the epiphyseal tethering plate for distal femur according to another embodiment of the invention.

### DETAILED DESCRIPTION OF THE INVENTION

Referring now to the figures, it is observed that the invention consists of a novel device for the rotational correction of bone malformations.

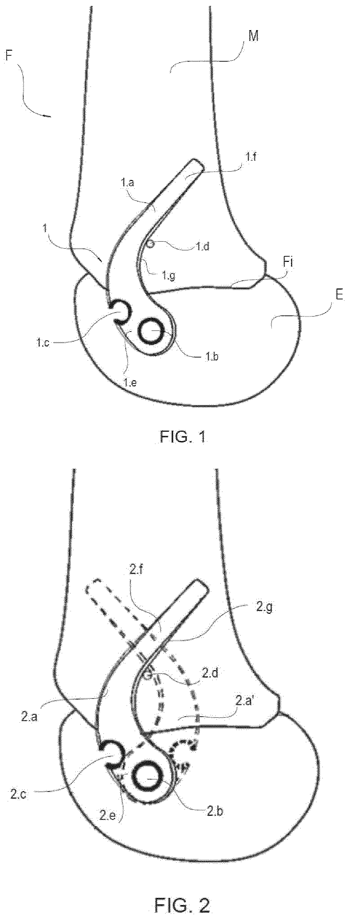

As can be seen in Figure 1, according to a sagittal view of a left distal femur, indicated in general with the reference F and having a zone E that is the epiphysis, and a zone M that is the metaphysis, both areas already explained above. It is important to clarify that, even when a femur is shown, the invention is applicable to the correction of any human or animal bone with a rotating disorder.

The device, indicated with the general reference 1, according to an embodiment of the invention, comprises a generally angled plate 1.a, preferably curved, having a first tethering end portion 1.e intended to be fixed to at least one of the bone zones E or M, and a second free end 1.f portion, or metaphyseal portion, intended to be free, i.e., it is not attached to the other zone E or M of the bone F. According to the terminology used in this description, if the first end portion 1.e is intended to be tethered or fixed to the epiphysis it can be called the epiphyseal tethering portion, and if the second end portion 1.f is intended to be free on the metaphysis it can also be called metaphyseal extension.

The device 1 also comprises a guide-stop 1.d that is attached to the bone area E or M, wherein the end 1.e of the plate 1.a. is not fixed. That is, if the plate is fixed to zone E or epiphysis, defining the aforementioned epiphyseal tethering, the guide-stop 1.d is fixed to zone M or metaphysis and is intended to define a guide support for the plate 1.a along an edge 1.g of the plate.

The plate 1.a features in its first end portion 1.e at least one hole 1.b intended to receive a fixing screw, not shown because it can be any of those well known in art. If a single screw applied in the hole 1.b is used, the type of screw and fixing should ensure the fixing against rotation of the plate 1.a.

Alternatively, the plate 1.a may include a second hole 1.c, to receive a second fixing screw, which is also not shown because it can of any type known in art. The hole 1.c can be fully closed or opened, as illustrated, because the screw placed in the hole 1.c is not intended to fix plate 1.a to the bone, which is the function of the screw applied in the hole 1.b, but its function is to prevent the rotation to the left, as shown in the figure, of plate 1.a, as will be explained below. For this same reason, hole 1.c can be simply a groove or recess in the plate or even none of these shapes is required in the plate profile.

As it is known, bone growth originates in the physis then, over time, the metaphysis extends axially away from the epiphysis. According to the invention, this extension of the bone is used to generate a guided relative rotation between the epiphysis, the physis and the metaphysis, in the desired sense, aimed at correcting the malalignment of, for example, the lower limbs of an individual, usually a child or teenager who is in their growth stage.

That is, this rotation is generated by means of the device of the invention taking advantage of the extension, i.e., growth, of the bone. With the first end portion 1.e fixed to the epiphysis E and with the guide-stop 1.d fixed to the metaphysis M, when bone is generated away from the epiphysis, guide-stop 1.d axially moves away from the first end portion 1.e of plate 1.a which is fixed to epiphysis E and is prevented from rotating. Given this arrest of rotation, the guide-stop 1.d is supported against the edge 1.g of the plate and as it moves axially away from hole 1.b it moves rotationally to the right according to the direction of Figure 1. Edge 1.g, by forming an angle with the first end portion 1.e, forces the rotational displacement of the guide-stop to the right of the figure and thus forces the metaphysis to rotate with respect to the epiphysis.

There is thus a torsional effect between epiphysis and metaphysis that generates the relative rotation between them. The support between the guide-stop and the fixing plate does not hold together any fixation that would represent a hindrance or lock or arrest of the axial distraction of the parts. In the worst-case scenario, which indeed can be expected and calculated, the guide-stop will have shifted axially so much that it will have exceeded the length of the end portion 1.f and will be out of contact with the guiding edge 1.g of plate 1.a. Thus, the birth malalignment of the individual is corrected. Periodic imaging monitoring control the correction, for example, to determine when it should be stopped.

Unlike the known techniques that fix plates or wires diagonally to the epiphysis and metaphysis, the present device is fixed only to one of them and its big advantage is as follows. When a certain point of growth is reached, i.e., a point of distance between E and M, a traditional plate or wire that had been fixed to the two zones E and F diagonally, due to the aforementioned distance, is disposed in an axial aligned form by the rotation generated between E and M. It is at this point wherein it starts to work as a stop or hindrance of further extension or growth of the bone and surgery must be performed to remove the plates or wire.

Unlike the above, plate 1.a forces the relative rotation between E and M but, by not being fixed on the other side, in this case M, it allows growth without any limitation. This concept explained in relation to the embodiment of the device of Figure 1 is extensive for all the remaining embodiments illustrated in Figures 2 through 13. In the different embodiments, the shape of the plates and their tethering or fixing are changed, as well as the shape of the guide-stops and their fixation. The epiphyseal sector or first tethering portion 1.e of the plate may preferably use screws of the type known as locked screws. The epiphyseal anchor in 1.b of these plates can be with one or more screws, preferably two screws, preferably screws that are locked or not, cannulated, or not.

Briefly, device 1 of the invention generates an internal rotating effect of the distal femur during physeal growth and the guide-stop with the screw or metaphyseal bar (1.d) does not prevent axial extension of the bone. The advantage of this system is that having no metaphyseal tethering but a guide-stop that, when moving axially, slides against the plate, can generate a rotating correction of the bone without a physeal arrest.

The fixation of the epiphyseal tethering, i.e., the first end portion 1.e of the plate, can be provided by screws which are locked or not, not shown because they are conventional, as well as any other form of anchoring that provides an epiphyseal fixation and which leaves the metaphyseal extension or second end portion 1.f of the plate free.

In a preferred form of use of the plate of the invention, the corrective device, to work more effectively, comprises a combination of double plate, i.e., an internal and an external plate, of the femur, as will be seen in the following Figures in dashed lines.

As shown in Figure 2, the plate shown in solid-lines is on this side of the bone while the plate that appears in dash-lines is disposed on the opposite side. This convention in the illustration of the plates is repeated in the rest of the figures that illustrate the use of two plates. When using two plates, one on each side of the femur, the metaphyseal extension or second end portion 1.f of the plates is disposed in the opposite direction, see Figure 2 as an example, and has the same angle of inclination with respect to the epiphysis E and physis Fi, which can be variable, for example, 60° of inclination with respect to the physis Fi. This metaphyseal extension or second end portion 1.f may be solid or it may comprise a central recess open to the free end or it can be closed, which can accommodate the guide-stop, as will be seen in the embodiments described below.

Figure 2 shows a sagittal view of a left distal femur, to which the device of the present invention is fixed, which, according to this embodiment, comprises a double plate, i.e., an internal plate 2.a and an external plate 2.e, which are symmetrical and can share the most central anchor hole 2.b or not if fixation is intended with a Kirschner wire locked on both sides on the plate with a double locking screw, all of these components, wires, screws, commonly used in the art. The guide-stop of the invention can be a bicortical bar or screw 2.d which must protrude on both sides of the bone to generate the desired stop on the plates and the rotary sliding effect. Even though in this Figure the reference numbers begin with 2, the parts of the plates are the same and equivalent to those in Figure 1. The reference numbers of the following figures will begin with the number of the corresponding figure.

Both internal and external plates can be attached with a symmetrical epiphyseal tethering with a locked KW with double locking head. Depending on the direction of metaphyseal extension or end portion 2.f of the internal and external plate, the effect of the metaphyseal stop will generate an internal or external rotation of the bone.

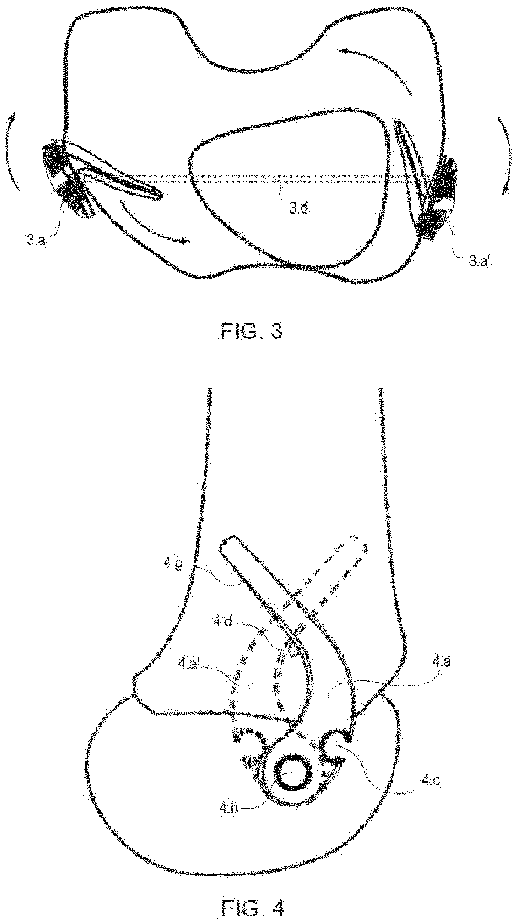

Figure 3 shows an axial view of a left femur with the internal plate 3.a and the external plate 3.a', similar or equal to those in Figures 1 and 2, wherein the guide-stop comprises a metaphyseal screw or bar 3.d that protrudes on each side of the cortical layer to define at its ends the guide-stops on which it takes place the sliding of the plates and generate the rotating effect on the plates.

Figure 4 also shows a sagittal view of a distal left femur, with internal plate 4.a and external plate 4.a', which mirror each other to generate an external rotation of the distal femur with the physeal growth and metaphyseal guide-stop 4.d.

The guide-stop, preferably fixed to the metaphysis, can comprise a screw or bicortical bar, parallel to the physis, that protrudes from the medial and lateral part of the cortical layer to be able to stop the end portions or metaphyseal extensions of both plates and that with physeal growth it moves away from the physis.

The design of the plates can vary as well as their mirror arrangement for distal femur, depending on whether the arrangement will be in the right or left femur and also whether the desired effect will be an external rotation or internal rotation of the bone during the physeal growth.

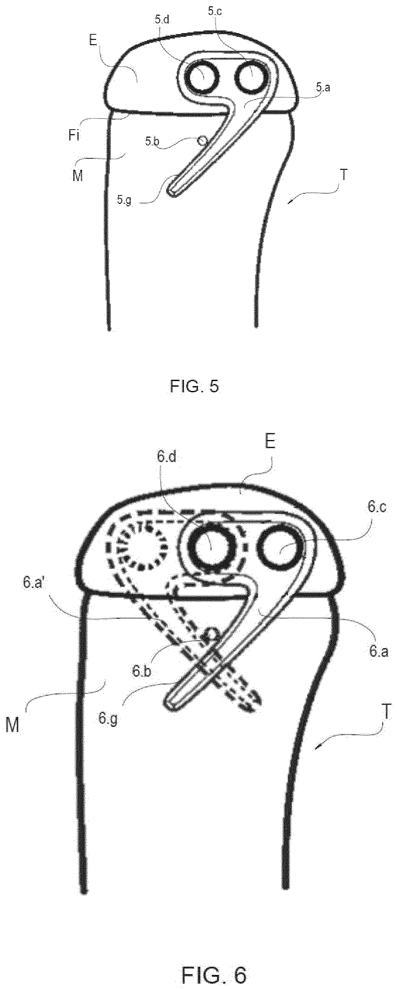

According to another embodiment of the invention, Figure 5 illustrates a sagittal view of a left proximal tibia T with a plate 5.a that has a design in form of a "7" and wherein its portion of epiphyseal tethering includes two holes 5.c and 5.d intended to receive screws and be fixed with the considerations with respect to the previous embodiments of Figures 1 to 4, when the device of the invention for femoral use was described.

Figure 6 illustrates two plates such as those in Figure 5, internal plates 6.a and external plates 6.a' also fixed in a left proximal tibia to generate an internal rotation with the guide-cap 6.d fixed to the metaphysis, with the same considerations of the previous embodiments established for distal femur.

For proximal tibia, the design of the plate in the form of a "7" is mounted in a mirroring arrangement, also depending on whether it is the right or left tibia, as well as whether the desired effect is an external or internal rotation of the bone during the physeal growth.

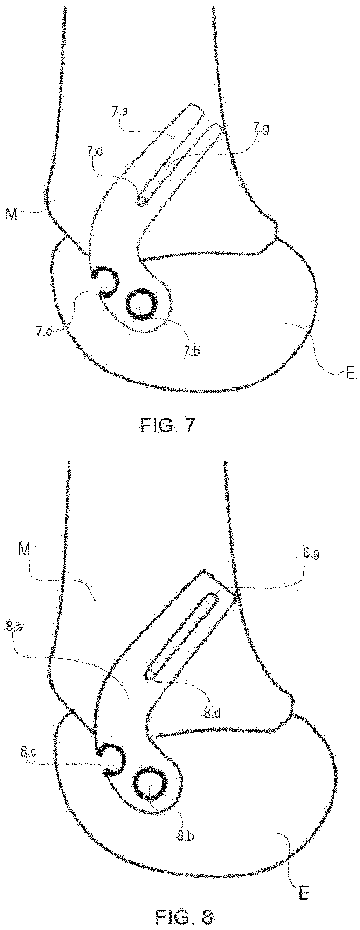

According to yet another embodiment of the invention, Figure 7 illustrates a plate 7.a with its epiphyseal tethering portion and its metaphyseal extension installed on a femur, wherein the metaphyseal extension presents a central slot or recess 7.g open in its distal sector to accommodate and guide the guide-stop 7.d.

According to yet another embodiment of the invention, Figure 8 illustrates a plate 8.a with its epiphyseal tethering portion and its metaphyseal extension installed on a femur, wherein the metaphyseal extension presents a central slot or recess 8.g closed in its distal sector to accommodate and guide the guide-stop 8.d.

These central slots 7.g and 8.g, with at least one of their inner edges, are equivalent to the guiding edge 1.g to 13.g and 15.g of the embodiments of Figures 1 to 13 and 15.

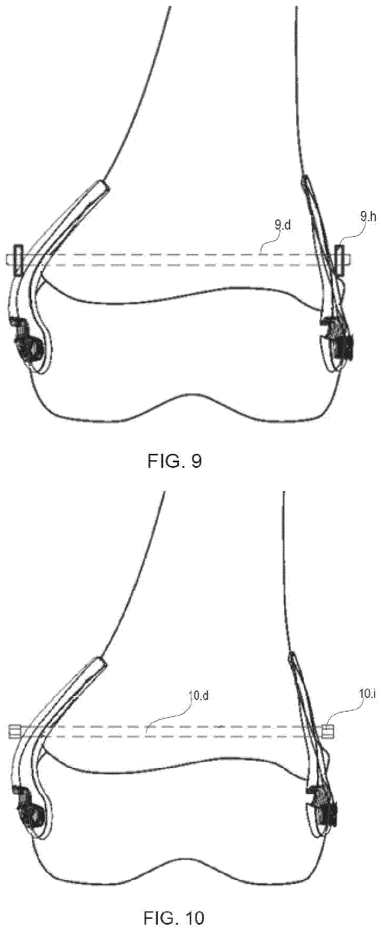

Figure 9 illustrates a preferred shape of the guide-stop that in this case comprises a simple cannulated screw 9.d that, when protruding from both sides of the cortical layer, defines, on each side, the guide-stop itself on which will abut the edges of the plates to be guided axially and rotationally. This screw can support two threaded washers 9.h to generate a side stop of the plate and prevent the system from being released and also to not leave the end of the screw free, which can generate injury to the soft parts or discomfort.

According to another embodiment, Figure 10 shows that the guide-stop is formed by a bicortical bar 10.d that, at both ends, has a faceted shape 10.i, for example, similar to an "Allen" key, for its removal by either of the two ends when it is necessary to loosen or remove the device of the invention.

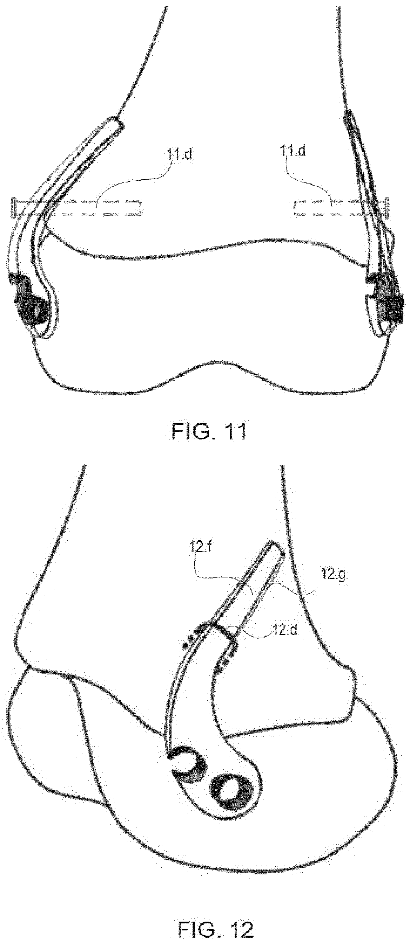

According to another embodiment, Figure **11** shows that the guide-stop is formed by two screws 11.d, independent of each other on each side of the bone.

According to another embodiment, Figure 12 shows that the guide-stop is formed by a staple 12.d inside of which the second end portion 12.f extends and slides to allow free axial extension and provoke the desired rotation.

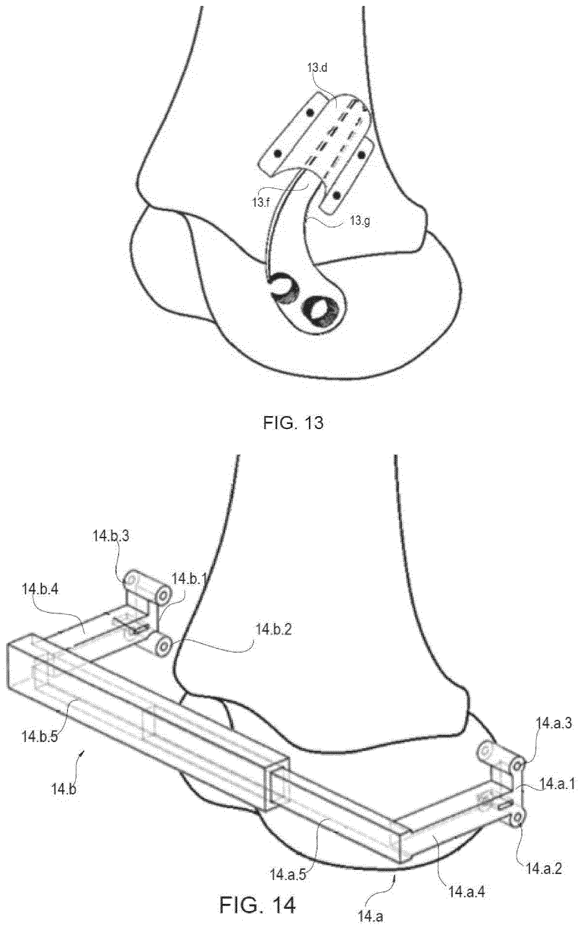

According to yet another embodiment, Figure 13 shows that the guide-stop is formed by a sleeve or jacket 13.d inside of which the second end portion 13.f extends and slides to allow free axial extension and provoke the desired rotation.

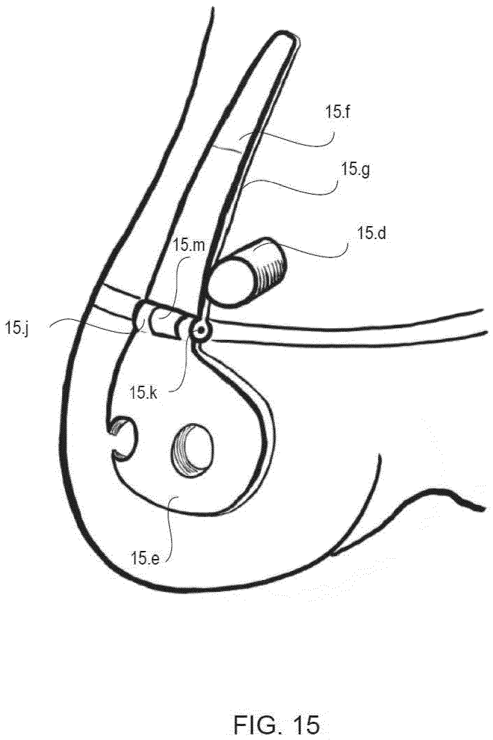

Figure 15 illustrates another embodiment of the invention and shows a sagittal view of the end of a long bone on which the device of the invention is fixed. In this alternative the fixing plate comprises the two portions that have been seen in the rest of the figures, a first tethering end portion 15.e and a second free end portion 15.f. The improvement provided in this embodiment is that the end portions 14.e and 15.f are joined together through a hinge that has hinge end parts 15.j and a hinge central part 15.m. Preferably the parts 15.j are integral with the first tethering end portion 15.e and the hinge central part 15.m is integral with the second free end portion 15.f, however, these parts could be inverted so that the parts 15.j are integral with the second end portion 15.f and the hinge central part 15.m is integral with the first tethering end portion of 15.e. This construction allows mobility in the frontal plane of this second end portion or free extension 15.f, which determines that it accommodates the accompanying metaphyseal diameter during the effect of rotary growth, avoiding it being an arrest element thereof. When installed, once portions 15.e and 15.f have been arranged in their desired position, parts 15.j and 15.m of the hinge are locked or fixed in position either by pressure, or by friction, or tooths inside parts 15.j and 15.m which can be tightened by an interior screw 15.k.

It is important to note that even though the plates have been illustrated with their epiphyseal tethering or first end portion 1.e fixed to the epiphysis and the metaphyseal extension or second end portion 1.f of the plate fixed to the metaphysis, this assembly could be reversed, which means that the first end portion 1.e could be fixed to the metaphysis and the second end portion 1.f could be fixed to epiphysis if the anatomy of the bone so permits. Even if at the time of removal of the device some discomfort may occur, this inverted placement is possible and is contemplated within the scope of the invention.

Also, according to the invention, a positioning/placement guide is provided, illustrated in Figure 14, and comprising an internal part 14.a and an external part 14.b. The internal part consists of a sector 14.a.1 designed to accommodate the shape of the plate, which has two cylinders for threading Kirschner wires, a radiopaque one represented in the centralizer hole of the first end portion, for example, portion 1.e of Figure 1, an epiphyseal hole of plate 14.a.2 and the other cylinder 14.a.3 where the second end portion 1.f or metaphyseal extension of the plate begins. This section of the interior part of the guide 14.a.1 that has the morphology of the plate comprises an arm of suitable form as a continuation, preferably rectangular 14.a.4, which features an arm also rectangular 14.a.5 as a continuation which will be coupled telescopically, or otherwise, to the horizontal hollow arm 14.b.5 of the exterior part 14.b of the guide. The external part of the guide also consists of a sector 14.b.1 designed to accommodate the shape of the plate, which has two cylinders for threading Kirschner wires, a radiopaque one represented in the centralizer hole of the first end portion 1.e, an epiphyseal hole of plate 14.b.2 and the other cylinder 14.b.3 where the second end portion, for example, portion 1.f of Figure 1, or metaphyseal extension of the plate begins. This section of the interior part of the guide 14.b.1 that has the morphology of the plate comprises an arm of suitable form as a continuation, preferably rectangular 14.b.4, which features the arm 14.b.5 of the inner part of the guide as a continuation after the metaphyseal Kirschner wire is threaded by the two cylinders of the guide, interior cylinder, and exterior cylinder.

The placement guide in Figure 14 has been developed to carry out a surgical implant technique, also according to the invention, for the correct positioning of both plates of the device, for example, the plates in Figures 1 to 13, and its correct operation with the metaphyseal guide-stop.

### Preferred surgical implant method

The preferred method or technique, according to the invention, to implant the device of the invention, comprises the following steps:
Step 1: A metaphyseal Kirschner wire is placed parallel to the physis E on the front of the bone, for example, 1 cm at metaphyseal level and in the center of the bone in the profile, seeking correct positioning with the use of the image intensifier.
Step 2: The placement guide is assembled on the Kirschner wire, through the cylinder 14.a.2, which allows to locate the correct positioning of the epiphyseal Kirschner wire.
Step 3: An epiphyseal Kirschner wire is placed through the guide and then the guide is removed.
Step 4: Milling is performed with a cannulated bit through the metaphyseal wire and a bicortical cannulated screw is placed. This is placed from the side in front of the plate, so that at the time of the rotation it is positioned upstream and facilitates its removal.

This bicortical screw is positioned so that it protrudes half a centimeter on each side of the bone, to generate the metaphyseal guide-stop, for example, 1.d of Figure 1 or the stop of the remaining embodiments.

Step 5: An epiphyseal approach of 3 cm is performed, internal and external, centered on the Kirschner wire for the correct sliding of the epiphyseal plate, for example, plate 1.a, the plate slides into contact with the second end portion 1.f, or metaphyseal extension, with the guide-stop already installed.

To facilitate the sliding of the plate, a threaded guide sleeve can be placed in the central locking hole and thus using it as a handle.

Step 6: The epiphyseal Kirschner wire is removed, the plate is placed with the centralizer hole 1.n where the Kirschner wire was located and milling is performed through the locking sleeve, placing the locked centralizer screw through the plate 1.a, then the second locked screw is placed on each side. It is closed by planes performing a correct washing and hemostasis and the final positioning of the device is controlled with an image intensifier.

### Method of placement of the guide

Also, according to the invention, the method or technique of using the positioning or placement guide comprises the following steps:
Step 1: Threading the metaphyseal hollow cylinder 14.a.3 and 14.b.3 of the interior and exterior part of the guide 14 to the metaphyseal Kirschner wire that is positioned in the bone.
Step 2: assembling the interior horizontal 14.a.5, solid rectangular, and exterior hollow rectangular 14.b.5 extensions of the guide.
Step 3: positioning of the placement guide in the profile, using the image intensifier to place the radiopaque epiphyseal hollow cylinder 14.a.2 and 14.b.2 in the center of the epiphysis, which allows to place the epiphyseal Kirschner wire in its correct position, then removing the guide.

The placement guide allows to determine the distance and orientation of placement of the epiphyseal Kirschner wire.

## Claims

1. Device for the correction of rotational malalignment syndrome in an individual suffering from the syndrome, wherein the device comprises:
At least one fixing plate (1.a) having a first tethering end portion (1.e) that has means of fixation (1.b-1.c) to attach to a first zone (E, M) of a bone, and a second free end portion (1.f) designed to extend over a second zone (E, M) of the bone,
at least one guide-stop (1.d) designed to be fixed in that second zone (E.M) of the bone, and
at least one guiding edge (1.g) in that second free end portion (1.f) for guided support against said guide-stop (1.g).

2. A device according to claim 1, wherein said means for fixing the fixing plate comprise at least one hole designed to receive a screw.

3. A device according to claim 2, wherein said means of fixation comprise two holes.

4. A device according to claim 2, wherein said means of fixation comprise at least said one hole and a cavity on an edge of said plate opposite said at least one guiding edge.

5. A device according to any one of the preceding claims, wherein said plate is curved.

6. A device according to any one of claims 1 to 4 wherein said first and second end portions of the plate form an angle between 30 and 70 degrees.

7. A device according to any one of the preceding claims, wherein said plate and said guide-stop are made of a material selected from the group comprising stainless steel, titanium, plastic, polymer, surgical steel, metal, biocompatible material and resorbable material.

8. A device according to any of the preceding claims, wherein said at least one fixing plate comprises two plates designed to be implanted each one on opposites sides of the bone.

9. A device according to any one of the preceding claims, wherein said at least one guiding edge is an external edge of the fixing plate, in said second free end portion.

10. A device according to any one of claims 1 to 8, wherein said at least one guiding edge is an internal edge in an open central recess extending along the second free end portion of the fixing plate.

11. A device according to any one of claims 1 to 8, wherein said at least one guiding edge is an internal edge in a closed central recess extending along the second free end portion of the fixing plate.

12. A device according to any one of the preceding claims, wherein said guide-stop comprises a medium selected from the group consisting of a bar, at least one screw, at least one staple, a pocket, a cannulated bicortical screw, a non-cannulated bicortical screw, a completely threaded screw, a partially threaded screw, a self-tapping screw, a self-piercing screw, a blunt tip screw, a bar with faceted ends.

13. A device according to any one of claims 1 to **11,** wherein the guide-stop is a bicortical bar configured to protrude from at least one of the two cortical layers.

14. A device according to any one of claims 1 to **11,** wherein the guide-stop is a mono or bicortical screw configured to generate a stop on the fixing plate, for which the screw protrudes from said cortical layer.

15. A device according to any of claims 1 to **11,** wherein the guide-stop is a cannulated screw, configured to protrude from both cortical layers and then be threaded by a cerclage wire or cable that surrounds the metaphyseal extension of the plate that is in contact with the tip of the screw and this cerclage is fixed to the head of the screw.

16. A device according to any one of the preceding claims, wherein said first tethering end portion and said second free end portion are joined together through a hinge.

17. A device according to any one of the preceding claims, wherein said first zone (E, M) of the bone is the epiphyseal zone of the bone and said second zone (E, M) of the bone is the metaphyseal zone of the bone.

18. A positioning guide for the installation of the device according to claim 1, comprising an internal part 14.a and an external part 14.b. movable together, wherein each of the internal and external parts has a sector 14.a.1, 14b.1 designed to accommodate the shape of the fixing plate, said sector comprising two cylinders for the threading of installation wires, these sectors being fixed to the respective arms 14.a.4 and 14.b.4 which in turn join arms 14.a.5 and 14.b.5 that are connected telescopically to each other.

19. A method of placing the positioning guide of claim 18, comprising the steps of:
Step 1: Threading, through one of the cylinders of the internal and external parts of the guide respectively, a metaphyseal Kirschner wire previously positioned in the bone;
Step 2: assembling the arms that are attached telescopically;
Step 3: positioning of the placement guide in the profile, using the image intensifier to place said one of the cylinders, which is a radiopaque epiphyseal hollow cylinder, in the center of the epiphysis, which allows to place the epiphyseal Kirschner wire in its correct position, and then removing the guide.

20. A method of installation of the device according to claim 1, comprising the following steps:
Step 1: placing a metaphyseal Kirschner wire parallel to the physis E on the front of the bone, for example, 1 cm at metaphyseal level and in the center of the bone in the profile, seeking correct positioning with the use of the image intensifier;
Step 2: assembling the placement guide on the Kirschner wire, through the cylinder, which allows to locate the correct positioning of the epiphyseal Kirschner wire;
Step 3: placing an epiphyseal Kirschner wire through the guide and then removing the guide;
Step 4: milling with a cannulated bit through the metaphyseal wire and placing a bicortical cannulated screw, from the side in front of the plate, so that at the time of the rotation it is positioned upstream and facilitates its removal, positioning the bicortical screw so that it protrudes half a centimeter on each side of the bone, to generate the metaphyseal guide-stop;
Step 5: performing an epiphyseal approach of 3 cm, internal and external, centered on the Kirschner wire for the correct sliding of the epiphyseal plate, sliding the plate into contact with the second end portion or metaphyseal extension, with the guide-stop already installed, the sliding of the plate can be facilitated by placing a threaded guide sleeve in the central locking hole and thus using it as a handle;
Step 6: removing the epiphyseal Kirschner wire, positioning the plate with the centralizer hole where the Kirschner wire was located and milling through the locking sleeve, placing the locked centralizer screw through the plate, then placing the second locked screw on each side, closing it by planes performing a correct washing and hemostasis and controlling with an image intensifier the final positioning of the device.
